# EUROPEAN PATENT SPECIFICATION

(11) **EP 4 577 161 B1**
(45) Date of publication and mention of the grant of the patent: **08.07.2026**
(21) Application number: 23758188.9
(22) Date of filing: 01.08.2023
(51) Int. Cl.: A61F 5/443

(54) **WATER RESISTANT CONNECTION FOR OSTOMY LEAKAGE DETECTION SYSTEM**
WASSERFESTE VERBINDUNG FÜR STOMA-LECKAGE-ERKENNUNGSSYSTEM
RACCORD RÉSISTANT À L'EAU POUR LE SYSTÈME DE DÉTECTION DES FUITES DES STOMIES

(30) Priority: 23.08.2022 US 202263400238 P
(43) Date of publication of application: 02.07.2025
(73) Proprietor: Hollister Incorporated, Libertyville, IL 60048 (US)
(72) Inventor: CARLSSON, Jonas P., Libertyville, IL 60048 (US); SMITH, Robert C., Libertyville, IL 60048 (US); RHINEHART, Grace E., Libertyville, IL 60048 (US); SMITH, Anthony B., Libertyville, IL 60048 (US); JAFFE, Elise, Libertyville, IL 60048 (US)
(74) Representative: FRKelly
(86) International application number: PCT/US2023/071434
(87) International publication number: WO 2024/044455

(56) References cited:
- WO-A1-2022/020841
- CN-A- 109 785 591
- US-A1- 2020 383 821
- US-A1- 2021 212 855

## Description

### BACKGROUND

This disclosure is related to a leakage detection system for medical devices, and more particularly to a leakage detection system for ostomy devices.

An ostomy pouch system typically includes a pouch formed from opposing sidewalls defining an internal collection area, an inlet opening for receiving a stoma, and an ostomy appliance for attaching the pouch to a user. The ostomy appliance may include, for example, an ostomy barrier of a one-piece pouch system, which is attached to one of the pouch sidewalls proximate an inlet opening, or a faceplate for a two-piece pouch system configured to releasably engage a pouch, and a barrier ring. The ostomy appliance may include a skin barrier material for adhering to and sealing against user's peristomal skin surrounding the stoma.

The ostomy appliance may be susceptible to ostomy effluent leakage, and the seal formed between the skin barrier material and the user may weaken. Oftentimes, the user may be unaware of, or cannot easily assess, an extent of weakening in the seal. Thus, the user may not become aware of a weakened seal, and consequently, the ostomy effluent may leak through to an exterior of the ostomy appliance.

Leakage detection systems may be used to detect leakage in ostomy appliances. However, these systems may provide false detection of moisture when a user is in wet environments rather than when there is a leak. For example, false detection may occur when a user is showering and moisture penetrates internal conductive members.

CN109785591A discloses an ostomy bag leakage alarm device comprising a detecting device and an alarm device, wherein the detecting device comprise an insulating seat and conductive rings and induction mechanism disposed therein, the insulating seat is annular, an inner ring surface can be sleeved at the outer edge of a chassis of the ostomy bag, there are two conductive rings arranged at intervals, a plurality of induction mechanisms are evenly arranged on the inner ring surface of the insulating seat circularly, two conductive electrodes of the induction mechanisms are electrically connectedto a conductive ring respectively, and the two conductive electrodes are disconnected under a normal state and are connected when there is leakage; the alarm device comprises a housing and a power supply and an alarm actuator disposed in the housing, wherein two power supply ends of the alarm actuator are electrically connected to the negative pole of the power supply and a conductive ring of thedetecting device respectively, and the positive pole of the power supply is electrically connected to the other conductive ring of the detecting device. The above-mentioned ostomy bag leakage alarm device can timely discover the leakage of the chassis 3 of the ostomy bag, and remind the patient to handle in time, which is convenient and safe for use.

US2020383821A discloses a base plate for an ostomy appliance, the base plate (4) comprises a top layer (208) defining a base plate plane; a first adhesive layer (200) adapted to adhere the base plate (4) to peristomal skin of a user; an electrode assembly (204); and a monitor interface (207) configured to electronically connect with the electrode assembly (204), where the monitor interface (207) comprises a coupling part (210) configured to form a releasably mechanically and/or electronically coupling between the base plate (4) and a monitor device (6); the coupling part (210) is configured to engage and/or disengage with the monitor device (6) allowing the monitor device (6) to be coupled to the base plate by a motion in a direction corresponding to an acute angle of 45 degrees or less relative to the base plate plane.

US2021212855A1 discloses a monitor device and an ostomy system comprising a monitor device (6) and an ostomy appliance (2) is disclosed, the ostomy appliance including a base plate (4), the base plate having a first adhesive layer with a proximal side configured for attachment of the base plate to the skin surface of a user, the first adhesive layer having a stomal opening (18) with a center point, the monitor device comprising a processor and a sensor unit comprising a first sensor with a first sensor surface accommodated in a monitor device housing, the monitor device housing having a sensor opening in a proximal surface of the monitor device, the sensor opening forming at least a part of a sensor path from surroundings of the proximal surface to the first sensor surface.

WO2022020841A1 discloses an ostomy leakage detection system (10) includes a sensing accessory (12) and a monitor device (14). The sensing accessory includes sensors configured to detect ostomy effluent leakage under a skin barrier (20) of an ostomy pouch system (18). The monitor device is configured to communicate with the sensing accessory to receive ostomy effluent leakage data. The monitor device is also configured to engage with the sensing accessory and configured to be attached to the ostomy pouch system or to user's body via the sensing accessory.

Accordingly, it is desirable to provide an improved leakage detection system for ostomy
devices.

### BRIEF SUMMARY

The invention is defined by independent claim 1. Advantageous embodiments are defined by the dependent claims.

An ostomy leakage detection system is provided according to various embodiments.

In one aspect, an ostomy leakage detection system may include a wearable subsystem, a sensing accessory, a connector region, and at least one gasket. The wearable subsystem may include conductive members. The sensing accessory may be configured to detect moisture under a barrier. The connector region may extend from the sensing accessory and may be configured to connect the wearable subsystem to the sensing accessory. The least one gasket may be configured to provide a water-resistant connection at the connector region.

In an embodiment, the connector region may include connection points configured to receive the conductive members. In such an embodiment, at least one gasket may be located on the connector region and configured to surround the connection points. In another embodiment, the at least one gasket may be located on the sensing accessory and surrounds a connection point.

In an embodiment, the at least one gasket may include two gaskets located on opposite sides of the connector region.

In an embodiment, the at least one gasket may include silicone, polyethylene, or ethylene vinyl acetate.

In an embodiment, the at least one gasket may be located on the wearable subsystem and surrounds the conductive members.

In an embodiment, the at least one gasket may be located on a bottom housing of the wearable subsystem. In such embodiment, an additional gasket may be located on a top housing of the wearable subsystem.

In an embodiment, the at least one gasket may be located on a top housing of the wearable subsystem.

**In** an embodiment, an additional gasket may be located on a bottom housing of the wearable subsystem.

**In** an embodiment, the connector region may include a key member configured to align a connection between the connector region and the wearable subsystem.

**In** an embodiment, the wearable subsystem may include a locking system.

The foregoing general description and the following detailed description are examples only and are not restrictive of the present disclosure.

### BRIEF DESCRIPTION OF THE DRAWINGS

The benefits and advantages of the present embodiments will become more readily apparent to those of ordinary skill in the relevant art after reviewing the following detailed description and accompanying drawings, wherein:
FIG. 1 is a perspective illustration of a wearable subsystem and sensor accessory according to an embodiment.
FIG. 2 is a perspective illustration of a wearable subsystem and sensor accessory according to another embodiment.
FIG. 3 is a perspective illustration of a wearable subsystem and sensor accessory according to another embodiment.
FIG. 4 is a perspective illustration of a wearable subsystem and sensor accessory according to another embodiment.
FIG. 5 is a perspective illustration of a wearable subsystem and sensor accessory according to another embodiment.
FIG. 6 is a perspective illustration of a sensor accessory according to another embodiment.
FIG. 7 is a schematic illustration of an ostomy system according to an embodiment.

### DETAILED DESCRIPTION

While the present disclosure is susceptible of embodiment in various forms, there is shown in the drawings and will hereinafter be described presently preferred embodiments with the understanding that the present disclosure is to be considered an exemplification and is not intended to limit the disclosure to the specific embodiments illustrated. The words "a" or "an" are to be taken to include both the singular and the plural. Conversely, any reference to plural items shall, where appropriate, include the singular.

The present disclosure provides an ostomy leakage detection system. The ostomy leakage detection system can be configured to detect ostomy effluent leakage under a skin barrier and alert a user. The ostomy leakage detection system can provide multiple benefits to the user. For example, the system can allow the user to intervene and change a skin barrier and/or ostomy pouch system before a leak progresses to cause embarrassment and inconvenience to the user. Further, the ostomy leakage detection system can assist in maintaining a user's skin health by alerting a leakage in its early stage to prevent prolonged skin exposure to ostomy effluent, which can lead to skin health complications. The ostomy leakage detection system can also support a user's emotional well-being by reducing anxiety associated with a risk of leakage. The ostomy leakage detection system may be applied to an ostomy barrier of a one-piece pouch system or a faceplate for a two-piece pouch system.

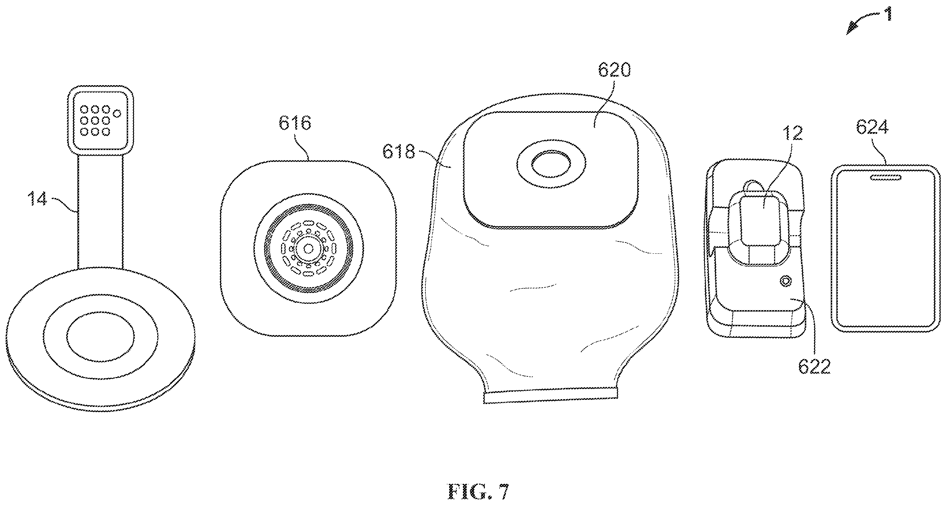

FIG. 7 illustrates an ostomy two-piece pouch system 1. In an embodiment, the ostomy system 1 generally includes a wearable subsystem 12, a sensing accessory 14, an ostomy barrier appliance 616, an ostomy bag 818, an ostomy barrier coupling member 620, a charging dock 622, and a mobile device 624.

In an embodiment, the ostomy leakage detection system may comprise four subsystems - the wearable subsystem 12, the sensing accessory 14, the charging dock 622, and a mobile application on the mobile device 624. The sensing accessory 14 may be provided as an accessory for an ostomy pouch system. The sensing accessory may include sensors for detecting the presence of ostomy effluent. The sensing accessory 14 may be configured to communicate leakage detection signals to the wearable subsystem 12.

The wearable subsystem may be configured to perform at least some processing of the leakage detection signals and alert a user of a leakage event. The wearable subsystem may be configured to communicate through wire or wirelessly with the mobile application. The mobile application may be a digital subsystem installed on a mobile device 624. The mobile application may be configured to process leak detection data and provide an alert or other information about an ostomy appliance to a user. The charging dock 622 may be configured to recharge and communicate with the wearable subsystem. The charging dock 622 may further provide alerts, for example, when the system is fully charged.

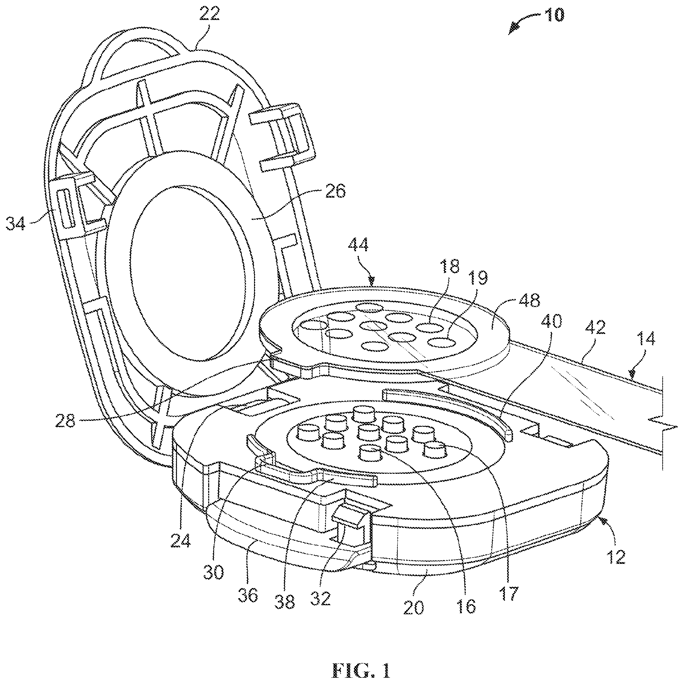

Referring now to the figures, FIGS. 1-5 show an ostomy leakage detection system 10 according to several embodiments. FIG. 1 is a perspective view of the ostomy leakage detection system 10 according to an embodiment. The ostomy leakage detection system 10 may generally include the wearable subsystem 12 and the sensing accessory 14. The sensing accessory 14 may be configured as an ostomy accessory that can be connected to an ostomy skin barrier on an ostomy barrier appliance, for example, an ostomy barrier of a one-piece pouch system or a faceplate for the two-piece pouch system (FIG. 7).

The wearable subsystem 12 may function as a relay between the sensing accessory 14 and a user or other subsystems of the leakage detection system 10, such as the mobile device 624. The wearable subsystem 12 may be configured to physically and electronically connect to the sensing accessory 14 and receive and analyze signals from the sensing accessory 14.

In an embodiment, the wearable subsystem 12 may generally comprise a hinged case, an embedded circuit board, a battery, a motor, an alignment member, and a gasket. The circuit board may include a conductive member 16 configured to contact terminal ends of electrical traces of the sensing accessory 14, such as the connection point 18. In this embodiment, the conductive member 16 comprising a plurality of raised conductive pads may be arranged generally in a center area of a bottom housing 20 of the wearable subsystem 12.

In an embodiment, the wearable subsystem 12 may include a hinged case comprising the bottom housing 20, a top housing 22, and a hinge 24 connecting the bottom housing 20 and the top housing 22. The hinge 24 allows the top housing 22 to rotate towards the top housing 22 about a fixed point on the hinge 24 and close the wearable subsystem 12. The wearable subsystem 12 may be configured to connect to the sensing accessory 14 and close on the sensing accessory 14 to keep a secure connection while in use.

In the embodiment of FIG. 1, the wearable subsystem 12 may further include a contact member 26, a key insert 30, a lock insert 32, a lock receiver 34, a release member 36, a first alignment member 38, and a second alignment member 40. The sensing accessory 14 may include a key member 28, an electrical connector 42, a connector region 44, one or more connection points 18, and a gasket 48.

The gasket 48 may be on the side of the connector region 44 that makes contact with the bottom housing 20. The gasket 48 can surround the conductive member 16. The gasket 48, when the wearable subsystem 12 is closed, is compressed by contact member 26 and the bottom housing 20 to form a water-resistant seal around the conductive member 16. A water-resistant seal is desirable for a user of the system because it will prevent false detection of moisture when in wet environments, such as while swimming, bathing, or showering. False detection occurs when fluid bridges conductive member 16 of the wearable subsytem 10, causing a drop in resistance similar to that when moisture is present at the sensor.

Locating the gasket 48 on sensing accessory 14 can be advantageous in that the number of use cycles for the gasket 48 may be limited. The sensing accessory 14 with the gasket 48 may be discarded after some use, while the wearable subsystem 12 can be used repeatedly. For example, the number of use cycles for the gasket 48 can be approximately 30, while the wearable subsystem 12 can be used for an extended period time, and perhaps even indefinitely.

In an embodiment, the gasket 48 may be formed from elastomeric materials, such as silicone, polyethylene, ethylene vinyl acetate, or other materials. The base material may be a closed-cell foam.

In an embodiment, conductive member 16 may include multiple conductive members grouped into multiple rows with one conductive member 17 located off-center and outside of the grouping. The connection point 18 may similarly include multiple connection points with a connection point 19 that is arranged in order to align and correctly connect with the corresponding conductive member 16.

In an embodiment, the outer edges of the connector region 44 can align with alignment members 38, 40. The alignment members 38, 40 can provide further support and alignment for the connector region 24 on the wearable subsystem 12.

In another embodiment, the alignment members 38, 40 can be one or more members that surround the conductive member 16.

In an embodiment, the first alignment member 38 can further include the key member 28 configured to align with key insert 30. The key member 28 and the key insert 30 can prevent the sensing accessory 14 from being connected upside down.

In an embodiment, the lock insert 32 may connect to lock receiver 34 when the wearable subsystem 12 is closed. The lock insert 32 and the lock receiver 34 are configured to lock the wearable subsystem 12 closed and keep a water-resistant seal. The release member 36 can be configured to release the lock insert 32 from the lock receiver 34 and open the wearable subsystem 12.

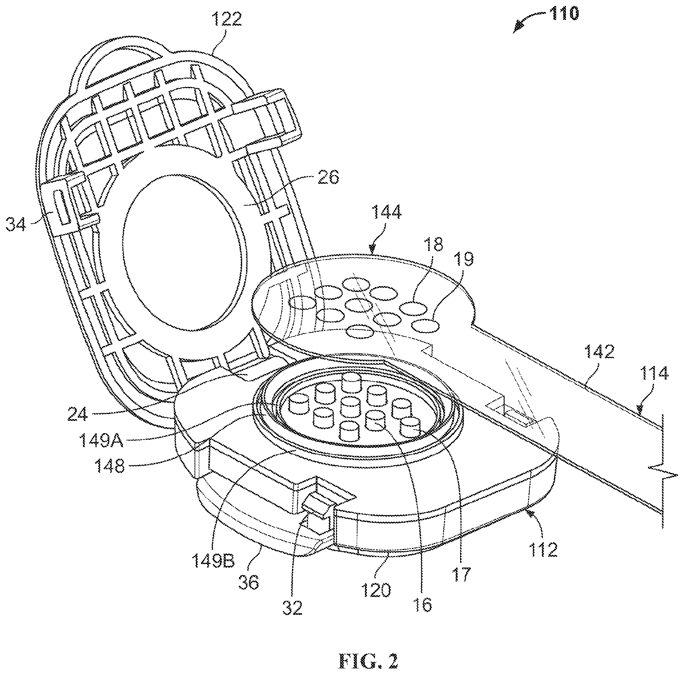

FIGS. 2-5 show an ostomy leakage detection systems 110, 210, 310, 410 according to several embodiments. The ostomy leakage detection systems 110, 210, 310, 410, may be configured similar to the ostomy leakage detection system 10 of FIG. 1, and may generally include a wearable subsystem 112, 212, 312, 412 and a sensing accessory 114, 214, 314, 414. The wearable subsystem 112, 212, 312, 412 may generally include a bottom housing 120, 220, 320, 420 top housing 122, 222, 322, 422 hinge 24, 224, 424 and conductive member 16. The sensing accessory 114, 214, 314 may generally include electrical connector 142, 242, 342, 442 connector region 144, 244, 344, 444 and connection point 18.

FIG. 2 is a perspective view of the ostomy leakage detection system 110. The ostomy leakage detection system 110, is similar to the ostomy leakage detection system 10 of FIG. 1 and generally includes the conductive member 17, the connection point 19, the hinge 24, the lock insert 32, the lock receiver 34, and the release member 36. In this embodiment, the leakage detecting system 110 can further include a gasket 148 located on the bottom housing 120 and surrounding the conductive members 16. The gasket 148 is configured to provide a water-tight seal around the conductive member 16 when the wearable subsystem 112 is closed.

In an embodiment, the gasket 148 on the bottom housing 120 may minimize material usage compared to a gasket on the sensing accessory. The gasket 148 on the bottom housing 120 may be reused indefinitely compared to a disposable sensing accessory that is used after a few cycles. The gasket 148 may be made out of materials that can withstand approximately 3000 use cycles over the lifetime of the wearable. In an embodiment, the gasket 148 can be applied in the form of an overmold through a screen printing or other printing process.

In an embodiment, bottom housing 120 can include surrounding rings 149A, 149B that are located on both side of the gasket 148 and can further help provide a water-tight seal.

In an embodiment, the conductive members 16 may be correctly connected to their corresponding connection points 18 based on the conductive member 17 and connection point 19 properly aligning. This prevents the sensing accessory 114 from being upside down or misaligned with the wearable subsystem 112.

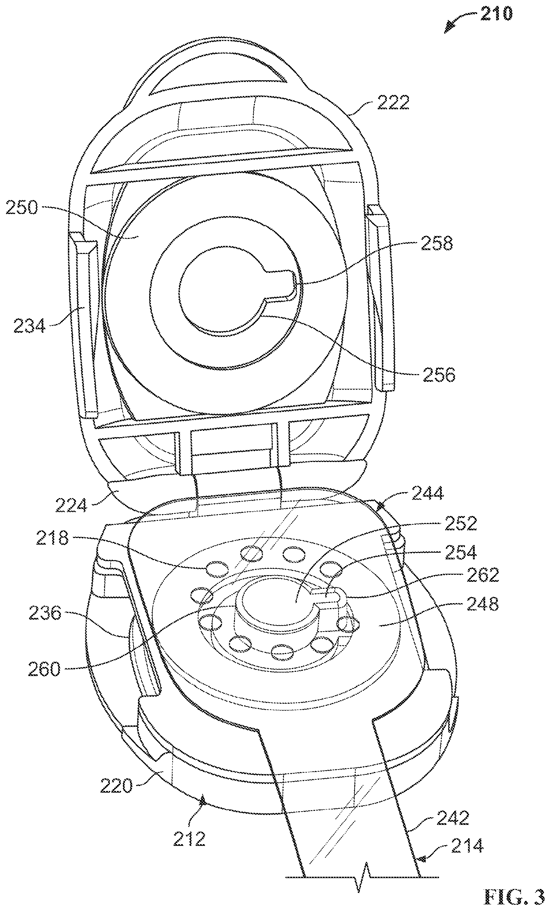

FIG. 3 is a perspective view of the ostomy leakage detection system 210 according to an embodiment. In this embodiment, the leakage detection system 210 further includes a first gasket 248 and a second gasket 250. The first gasket 248 may be arranged on the bottom housing 220 surrounding the conductive members (not shown). The second gasket 250 may be arranged on the top housing 222. The gaskets 248, 250 are configured to close into and sandwich the sensing accessory 214 when the top housing 222 is close on the bottom housing 220 to provide a water-tight seal around the conductive members.

In an embodiment, the bottom housing 220 can further include a center raised key member 252. The center raised key member 252 can include a key member 254. The top housing 222 can include a center alignment insert 256. The center alignment insert 256 can include a key insert 258. The sensing accessory 214 can include a center alignment opening 260. The center alignment opening 260 can include a key opening 262. The connector region 244 can be connected to the wearable subsystem 212 by placing the center raised key member 252 and the key member 254 through the center alignment opening 260 and the key opening 262. The key member 254 and the key opening 262 prevent the connector region 244 from being connected upside down or misaligned. The wearable subsystem 112 can be closed and the center raised key member 252 and the key member 254 align with the center alignment insert 256 and the key insert 258.

In this embodiment, the conductive members can be arranged in a circular grouping around the center raised key member 252 and surrounded by the gasket 248. The connection points 18 may similarly be arranged in a circular grouping around the center alignment opening 246.

In an embodiment, the wearable subsystem 112 can further include a lock receiver 234 and lock insert 236. The lock receiver 234 can be located on the top housing 222 and the lock insert 236 can be located on the bottom housing 220. The lock receiver 234 may connect to the lock insert 236 and keep the wearable subsystem 212 closed with a water-resistant seal.

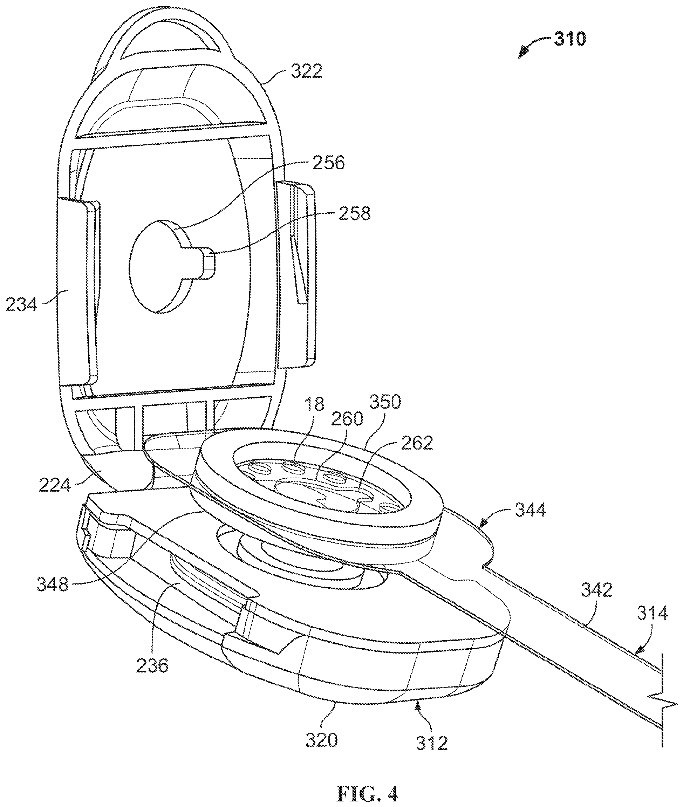

FIG. 4 is a perspective view of the ostomy leakage detection system 310 according to an embodiment. The ostomy leakage detection system 310, may be similar to the ostomy leakage detection system 210 of FIG. 3 and may generally include the hinge 224, the center raised key member 252, the key member 254, center alignment insert 256, the key insert 258, the center alignment opening 260, the key opening 262, the lock insert 236, and the lock receiver 254. In this embodiment, the leakage detecting system 310 can further include a bottom gasket 348 located on one side of the connector region 344 and a top gasket 350 located on an opposite side of the connector region 344. The bottom gasket 348 is configured to make contact with the bottom housing 320. The top gasket 350 is configured to make contact with the top housing 322. The gaskets 348, 350 surround the connection points 18, the center alignment opening 260, and the key opening 262. The gaskets 348, 350 are configured to provide a water-tight seal around the connection points 18 when the wearable subsystem 112 is closed.

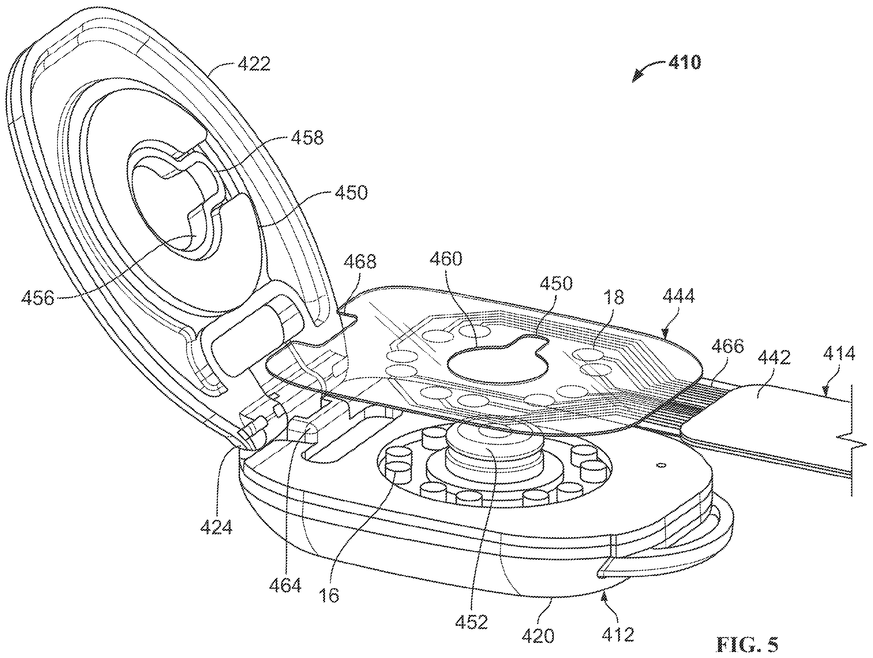

FIG. 5 is a perspective view of the ostomy leakage detection system 410 according to an embodiment. In this embodiment, the leakage detection system 410 can further include a gasket 450, a center raised key member 452, a key member (not shown), a center insert 456, a key insert 458, a center alignment opening 460, a key opening 462, a peripheral raised member 464, electrical leads 466, and an edge alignment 468. The gasket 450, the center raised key member 452 and the key member can be arranged on the top housing 422. The gasket 450 can surround the center insert 456 and the key insert 458. The center raised key member 452, key member, and the peripheral raised member 464 can be arranged on the bottom housing 420. The center raised key member 452 and key member can be surrounded by the conductive members 16. The center alignment opening 460, the key opening 462, the electrical leads 466, and an edge alignment 468 can be arranged on the sensing accessory 414. The center alignment opening 460 and the key opening 462 are surrounded by the connection point 18. The electrical leads 466 can provide an electrical connection between the wearable device 412 and the sensing accessory 414.

In an embodiment, the connector region 444 can be connected to the wearable subsystem 412 by placing the center raised key member 452 and the key member through the center alignment opening 460 and the key opening 462 and aligning the edge alignment 468 with the peripheral raised member 464. The key member and the key opening 462 prevent the connector region 444 from being connected upside down or misaligned. The edge alignment 468 and the peripheral raised member 464 prevent the connector region 44 from being misaligned and connected sideways. The wearable subsystem 412 can then be closed and secured.

In an embodiment, the gasket 430 can cover the conductive members 16 and the connection point 18 to provide a water-resistant seal when the wearable subsystem 412 is closed.

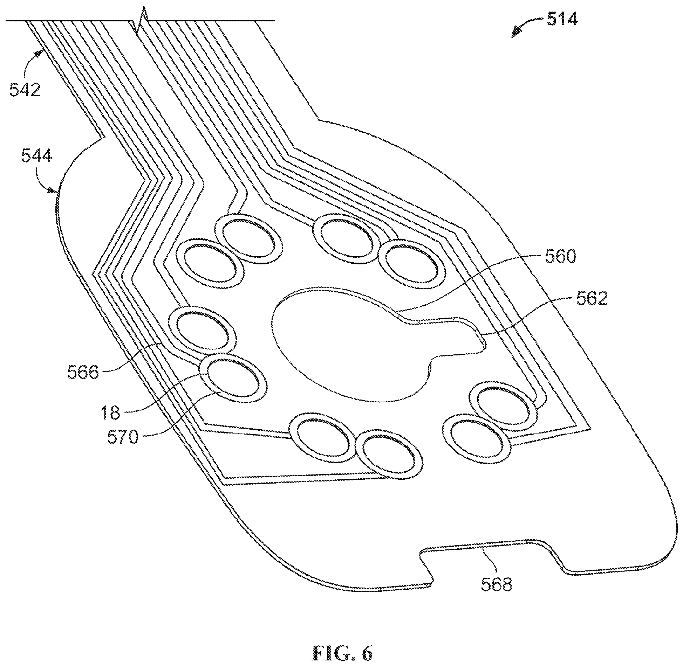

FIG. 6 is a perspective view of a sensor accessory 514 according to another embodiment. The sensing accessory 514 can include a connection point 18, an electrical connector 542, a connector region 544, a center alignment opening 560, a key opening 562, electrical leads 566, and an edge alignment 468. In this embodiment, the sensing accessory 514 can further include a gasket 570. The gasket 570 surrounds an individual connection point 18. The gasket 570 is configured to provide a water-tight seal around the connection between the conductive member 16 and the connection point 18 when the wearable device 12 is closed.

The electrical leads 466, 566 can connect the leakage detection circuitry in the ostomy barrier of the one-piece pouch system or the faceplate of the two-piece pouch system to the connection points 18.

In one or more embodiments, a water-resistance seal may prevent ingress of water when submerged under 1 meter for 30 minutes. This rating is sufficient to prevent false alerts during common user activities, such as swimming and bathing.

A simulated deflection test on the contact member 26 of the top housing 22 of FIG. 1 when the wearable subsystem 12 is closed was conducted. The deflection test simluated the load necessary to close and seal the wearable substem. The results show that the maximum deflection occurs away from the hinge and therefore, the design is suitable for low to maintain compression.

In an embodiment, the force-to-close range may be from 8.9 N and 44.48 N to 13.35 N to 22.25 N (2 and 10 lbf (pound-force) to 3 to 5 lbf). The distribution of the force across the plane of the wearable is arranged to keep the face substantially planar and maintain compression.

## Claims

1. An ostomy leakage detection system (10, 110, 210, 310, 410, 510) comprising:
a wearable subsystem (12, 112, 212, 312, 412) comprising conductive members (16, 17), a bottom housing (20, 120, 220, 320, 420), and a top housing (22, 122, 222, 322, 422) comprising a contact member (26);
a sensing accessory (14, 114, 214, 314, 414) configured to detect moisture under a barrier,
wherein the wearable subsystem is configured to connect to the sensing accessory and close on the sensing accessory to keep a secure connection while in use;
a connector region (44, 144, 244, 344, 444) extending from the sensing accessory and configured to connect the wearable subsystem to the sensing accessory; and
at least one gasket (48, 148, 248, 348, 448, 570) configured to provide a water-resistant connection at the connector region, wherein
the at least one gasket is located on the wearable subsystem and surrounds the conductive members, and the gasket, when the wearable subsystem is closed on the sensing accessory, is compressed by the contact member and the bottom housing to form a water-resistant seal around the conductive members.

2. The ostomy leakage detection (10, 110, 210, 310, 410, 510) system of claim 1, wherein the connector region (44, 144, 244, 344, 444) comprises connection points (18, 19) configured to receive the conductive members.

3. The ostomy leakage detection system (10, 110, 210, 310, 410, 510) of claim 1, wherein the at least one gasket comprises silicone, polyethylene, or ethylene vinyl acetate.

4. The ostomy leakage detection system (10, 110, 210, 310, 410, 510) of claim 1, wherein the at least one gasket is located on the bottom housing of the wearable subsystem.

5. The ostomy leakage detection system (10, 110, 210, 310, 410, 510) of claim 4, further comprising an additional gasket (250, 350, 450) located on the top housing (22, 122, 222, 322, 422) of the wearable subsystem.

6. The ostomy leakage detection system (10, 110, 210, 310, 410, 510) of claim 1, wherein the at least one gasket is located on the top housing of the wearable subsystem.

7. The ostomy leakage detection system (10, 110, 210, 310, 410, 510) of claim 6, further comprising an additional gasket located on the bottom housing of the wearable subsystem.

8. The ostomy leakage detection system (10, 110, 210, 310, 410, 510) of claim 1, wherein the connector region comprises a key member (28) configured to align a connection between the connector region and the wearable subsystem.

9. The ostomy leakage detection system (10, 110, 210, 310, 410, 510) of claim 1, wherein the wearable subsystem comprises a locking system (32, 34).

10. The ostomy leakage detection system (10, 110, 210, 310, 410, 510) of claim 2, further comprising leakage detection circuitry, and wherein the sensing accessory (14, 114, 214, 314, 414) further comprises electrical leads (466, 566) configured to connect the leakage detection circuitry and the connection points.

11. The ostomy leakage detection system (10, 110, 210, 310, 410, 510) of claim 1, wherein the wearable subsystem further comprises a hinge (24, 224, 424) connecting the bottom housing and the top housing.

## Patentansprüche

1. Stoma-Leckage-Erkennungssystem (10, 110, 210, 310, 410, 510), umfassend:
ein am Körper tragbares Teilsystem (12, 112, 212, 312, 412), umfassend leitfähige Elemente (16, 17), ein unteres Gehäuse (20, 120, 220, 320, 420) und ein oberes Gehäuse (22, 122, 222, 322, 422), das ein Kontaktelement (26) umfasst;
ein Erfassungszubehörteil (14, 114, 214, 314, 414), das dafür konfiguriert ist, Feuchtigkeit unter einer Barriere zu detektieren, wobei das am Körper tragbare Teilsystem dafür konfiguriert ist, eine Verbindung mit dem Erfassungszubehörteil herzuwerfen und sich auf dem Erfassungszubehörteil zu schließen, um eine sichere Verbindung während des Gebrauchs aufrechtzuerhalten;
einen Verbinderbereich (44, 144, 244, 344, 444), der sich von dem Erfassungszubehörteil erstreckt und dafür konfiguriert ist, das am Körper tragbare Teilsystem mit dem Erfassungszubehörteil zu verbinden; und
mindestens eine Dichtung (48, 148, 248, 348, 448, 570), die dafür konfiguriert ist, eine wasserfeste Verbindung an dem Verbinderbereich bereitzustellen, wobei sich die mindestens eine Dichtung an dem am Körper tragbaren Teilsystem befindet und die leitfähigen Elemente umgibt, und die Dichtung, wenn das am Körper tragbare Teilsystem auf dem Erfassungszubehörteil geschlossen ist, durch das Kontaktelement und das untere Gehäuse komprimiert wird, um eine wasserfeste Abdichtung um die leitfähigen Elemente herum zu bilden.

2. Stoma-Leckage-Erkennungs-(10, 110, 210, 310, 410, 510)-System nach Anspruch 1, wobei der Verbinderbereich (44, 144, 244, 344, 444) Verbindungspunkte (18, 19) umfasst, die dafür konfiguriert sind, die leitfähigen Elemente aufzunehmen.

3. Stoma-Leckage-Erkennungssystem (10, 110, 210, 310, 410, 510) nach Anspruch 1, wobei die mindestens eine Dichtung Silikon, Polyethylen oder Ethylenvinylacetat umfasst.

4. Stoma-Leckage-Erkennungssystem (10, 110, 210, 310, 410, 510) nach Anspruch 1, wobei sich die mindestens eine Dichtung an dem unteren Gehäuse des am Körper tragbaren Teilsystems befindet.

5. Stoma-Leckage-Erkennungssystem (10, 110, 210, 310, 410, 510) nach Anspruch 4, ferner umfassend eine zusätzliche Dichtung (250, 350, 450), die sich an dem oberen Gehäuse (22, 122, 222, 322, 422) des am Körper tragbaren Teilsystems befindet.

6. Stoma-Leckage-Erkennungssystem (10, 110, 210, 310, 410, 510) nach Anspruch 1, wobei sich die mindestens eine Dichtung an dem oberen Gehäuse des am Körper tragbaren Teilsystems befindet.

7. Stoma-Leckage-Erkennungssystem (10, 110, 210, 310, 410, 510) nach Anspruch 6, ferner umfassend eine zusätzliche Dichtung, die sich an dem unteren Gehäuse des am Körper tragbaren Teilsystems befindet.

8. Stoma-Leckage-Erkennungssystem (10, 110, 210, 310, 410, 510) nach Anspruch 1, wobei der Verbinderbereich ein Passelement (28) umfasst, das dafür konfiguriert ist, eine Verbindung zwischen dem Verbinderbereich und dem am Körper tragbaren Teilsystem auszurichten.

9. Stoma-Leckage-Erkennungssystem (10, 110, 210, 310, 410, 510) nach Anspruch 1, wobei das am Körper tragbare Teilsystem ein Verriegelungssystem (32, 34) umfasst.

10. Stoma-Leckage-Erkennungssystem (10, 110, 210, 310, 410, 510) nach Anspruch 2, ferner umfassend eine Leckageerkennungsschaltung, und wobei das Erfassungszubehörteil (14, 114, 214, 314, 414) ferner elektrische Zuleitungen (466, 566) umfasst, die dafür konfiguriert sind, die Leckageerkennungsschaltung und die Verbindungspunkte zu verbinden.

11. Stoma-Leckage-Erkennungssystem (10, 110, 210, 310, 410, 510) nach Anspruch 1, wobei das am Körper tragbare Teilsystem ferner ein Scharnier (24, 224, 424) umfasst, das das untere Gehäuse und das obere Gehäuse verbindet.

## Revendications

1. Système de détection des fuites des stomies (10, 110, 210, 310, 410, 510) comprenant :
un sous-système portable (12, 112, 212, 312, 412) comprenant des éléments conducteurs (16, 17), un boîtier inférieur (20, 120, 220, 320, 420) et un boîtier supérieur (22, 122, 222, 322, 422) comprenant un élément de contact (26) ;
un accessoire de détection (14, 114, 214, 314, 414) configuré pour détecter l'humidité sous une barrière, dans lequel le sous-système portable est configuré pour se connecter à l'accessoire de détection et se fermer sur l'accessoire de détection pour maintenir une connexion sécurisée pendant l'utilisation ;
une région de connexion (44, 144, 244, 344, 444) s'étendant depuis l'accessoire de détection et configurée pour connecter le sous-système portable à l'accessoire de détection ; et
au moins un joint (48, 148, 248, 348, 448, 570) configuré pour fournir un raccord résistant à l'eau au niveau de la région de connexion, dans lequel l'au moins un joint est situé sur le sous-système portable et entoure les éléments conducteurs, et le joint, lorsque le sous-système portable est fermé sur l'accessoire de détection, est comprimé par l'élément de contact et le boîtier inférieur pour former un joint résistant à l'eau autour des éléments conducteurs.

2. Système de détection des fuites des stomies (10, 110, 210, 310, 410, 510) selon la revendication 1, dans lequel la région de connexion (44, 144, 244, 344, 444) comprend des points de connexion (18, 19) configurés pour recevoir les éléments conducteurs.

3. Système de détection des fuites des stomies (10, 110, 210, 310, 410, 510) selon la revendication 1, dans lequel l'au moins un joint comprend du silicone, du polyéthylène ou de l'acétate de vinyle d'éthylène.

4. Système de détection des fuites des stomies (10, 110, 210, 310, 410, 510) selon la revendication 1, dans lequel l'au moins un joint est situé sur le boîtier inférieur du sous-système portable.

5. Système de détection des fuites des stomies (10, 110, 210, 310, 410, 510) selon la revendication 4, comprenant en outre un joint supplémentaire (250, 350, 450) situé sur le boîtier supérieur (22, 122, 222, 322, 422) du sous-système portable.

6. Système de détection des fuites des stomies (10, 110, 210, 310, 410, 510) selon la revendication 1, dans lequel l'au moins un joint est situé sur le boîtier supérieur du sous-système portable.

7. Système de détection des fuites des stomies (10, 110, 210, 310, 410, 510) selon la revendication 6, comprenant en outre un joint supplémentaire situé sur le boîtier inférieur du sous-système portable.

8. Système de détection des fuites des stomies (10, 110, 210, 310, 410, 510) selon la revendication 1, dans lequel la région de connexion comprend un élément clé (28) configuré pour aligner une connexion entre la région de connexion et le sous-système portable.

9. Système de détection des fuites des stomies (10, 110, 210, 310, 410, 510) selon la revendication 1, dans lequel le sous-système portable comprend un système de verrouillage (32, 34).

10. Système de détection des fuites des stomies (10, 110, 210, 310, 410, 510) selon la revendication 2, comprenant en outre un circuit de détection des fuites, et dans lequel l'accessoire de détection (14, 114, 214, 314, 414) comprend en outre des conducteurs électriques (466, 566) configurés pour connecter le circuit de détection des fuites et les points de connexion.

11. Système de détection des fuites des stomies (10, 110, 210, 310, 410, 510) selon la revendication 1, dans lequel le sous-système portable comprend en outre une charnière (24, 224, 424) reliant le boîtier inférieur et le boîtier supérieur.
